# DEMANDE DE BREVET EUROPEEN

(11) **EP 2 138 054 A1**
(43) Date de publication de la demande: **30.12.2009**
(21) Numéro de dépôt: 09163948.4
(22) Date de dépôt: 26.06.2009
(51) Int. Cl.: A23L 1/29, A61K 31/035, A61K 36/9068, A61K 36/9062, A61P 11/00

(54) **Extrait de plante pour la prévention ou le traitement du ronflement et/ou de l'apnée du sommeil**

(30) Priorité: 26.06.2008 FR 0854267
(71) Demandeur: Cariel, Léon, 75001 Paris (FR); Rabhi, Chérif, 91600 Savigny sur Orge (FR)
(72) Inventeur: Cariel, Léon, 75001 Paris (FR); Rabhi, Chérif, 91600 Savigny sur Orge (FR)
(74) Mandataire: Colombet, Alain André

(57) **Abrégé**

La présente invention concerne un nouvel extrait de plante et les compositions contenant cet extrait pour la prévention, la diminution ou la suppression du ronflement et/ou de l'apnée du sommeil. L'invention concerne plus particulièrement une composition comprenant au moins un terpène ou un terpénoïde et un extrait d'une plante de la famille des Zingiberaceae, de préférence choisie parmi les espèces *Alpinia galanga, Alpinia officinarum, Zingiber officinalis, Zingiber cassumunar* et *Zingiber zerumbet,* de préférence *Alpinia galanga,* l'extrait contenant des dérivés de phénylpropène et étant exempt de dérivés de type chavicol. Elle concerne aussi un procédé d'obtention d'un tel extrait comprenant une étape de macération en présence d'un solvant, puis une seconde étape dans laquelle l'extrait issu de la macération est soumis au reflux d'une solution aqueuse.

## Description

La présente invention concerne un nouvel extrait de plante et les compositions contenant cet extrait pour la prévention, la diminution ou la suppression du ronflement et/ou de l'apnée du sommeil.

L'apnée du sommeil est une condition sérieuse qui se traduit par des pauses respiratoires répétées pendant le sommeil. Ces pauses ou apnées durent habituellement de 10 à 30 secondes et peuvent se produire plusieurs fois pendant la nuit.

La forme la plus courante de l'apnée du sommeil est l'apnée obstructive du sommeil, qui se caractérise par un blocage des voies aériennes supérieures pendant le sommeil. Souvent, ce blocage se produit lorsque le tissu mou, au fond de la gorge, se relâche et se referme pendant la nuit. Des muscles de gorge relâchés, une voie aérienne étroite, une langue de grande taille ou un excès de tissus graisseux dans la gorge peuvent aussi bloquer le passage de l'air.

Pendant le sommeil profond, la fréquence cardiaque et la pression artérielle diminuent, ce qui permet au coeur de se reposer. Les épisodes de ronflement, de pauses respiratoires et d'étouffement pour retrouver son air, causés par l'apnée du sommeil, empêchent d'obtenir un sommeil de bonne qualité. Ces phénomènes peuvent être une source d'hypertension artérielle, de crise cardiaque et d'accident vasculaire cérébral.

Par ailleurs, le ronflement reste une gêne pour l'entourage.

Les méthodes de lutte contre le ronflement et l'apnée du sommeil sont essentiellement la chirurgie et l'administration de compositions de confort ou de médicaments.

On trouve ainsi dans le commerce des produits à base d'huile, parfois d'huile essentielle, éventuellement contenus dans un vecteur de type liposome ou corps gras destiné à retenir l'huile au niveau des tissus mous de la bouche et de la gorge.

WO03/080036 propose de prévenir, supprimer ou diminuer le ronflement et traiter l'apnée du sommeil à l'aide d'un vanilloïde, notamment choisi parmi la pipérine, les gingerols et les shogaols. Suivant un mode de réalisation, est employé un extrait de plante de la famille des Zingiberaceae.

Toutefois, un extrait préparé selon le procédé d'extraction décrit dans WO03/080036 s'est avéré impropre à une utilisation chez l'homme, la préparation s'avérant trop agressive pour l'estomac et pour une évaluation de son efficacité. La présente demanderesse a en réalité découvert que ce procédé antérieur conduit à un extrait contenant des dérivés de chavicol, lesquels s'avèrent très agressifs pour l'estomac.

Des procédés de production d'extraits végétaux à partir de plantes de la famille des Zingiberaceae ont aussi été décrits dans EP1071440 et WO 2005/044253.

EP1071440 décrit un extrait contenant de 2 à 99,5 % p/p d'un extrait ou concentré *d'Alpinia galanga,* contenant des composés choisis parmi des dérivés de type chavicol et des acétates et alcools dérivés des précédents, et de 0,5 à 98% p/p d'huile essentielle provenant d'Alpinia galanga. Ce document est très orienté sur la présence de dérivés de chavicol et notamment du 1'-acétoxyacétate de chavicol. Ces extraits sont utiles dans le domaine de l'immunomodulation.

WO 2005/044253 concerne lui la stimulation de la libido à l'aide de dérivés de chavicol ou de produits dérivés obtenus par réarrangement sigmatropique, et d'extraits d'Alpinia officinarum en contenant.

Devant l'absence dans la pharmacopée de traitement efficace et toléré de l'apnée du sommeil et du ronflement, l'intérêt de rechercher une composition efficace pour la prévention, la diminution ou la suppression du ronflement, et/ou de l'apnée du sommeil reste entier.

La demanderesse a découvert que le mélange de terpène ou terpénoïde e.g. huile essentielle et de certains extraits de plantes de la famille des Zingiberaceae, répond à ce besoin. Ces extraits ont subi un traitement transformant des dérivés de type chavicol en dérivés phénylpropène. La demanderesse a aussi découvert que les compositions répondant à cette définition étaient d'autant plus efficaces qu'elles répondaient en plus à des critères qualitatifs et quantitatifs qui seront abordés plus loin.

La présente invention a ainsi pour objet une composition comprenant au moins un terpène ou un terpénoïde et un extrait d'une plante de la famille des Zingiberaceae, l'extrait contenant des dérivés de phénylpropène, et étant exempt de dérivés de type chavicol. Parmi les plantes de la famille des Zingiberaceae, on peut mentionner notamment les espèces *Alpinia galanga, Alpinia officinarum, Zingiber officinalis, Zingiber cassumunar* et *Zingiber zerumbet.* Dans un mode préféré de l'invention l'extrait de Zingiberaceae est celui *d'Alpinia galanga.*

Les terpènes et terpénoïdes sont les constituants principaux des huiles essentielles. Suivant un mode de réalisation préféré, la composition comprend une huile essentielle, qui apporte tout ou partie des terpènes ou terpénoïdes présents dans la composition.

La notion d'huile essentielle est connue de l'homme de l'art. Il s'agit d'un liquide huileux, généralement de couleur jaune fonçant ou brun à la lumière, concentré et hydrophobe réunissant les composés aromatiques volatiles d'une plante. Les huiles essentielles peuvent être obtenues par les techniques de distillation ou d'extraction, par exemple distillation par entraînement à la vapeur, distillation par les solvants volatils, extraction par CO₂ supercritique.

Comme huile essentielle utilisable, on peut mentionner les huiles obtenues à partir de la cardamome ou d'une plante de la famille des Zingiberaceae, notamment choisie parmi les espèces *Alpinia galanga, Alpinia officinarum, Zingiber officinalis, Zingiber cassumunar* et *Zingiber zerumbet.*

Suivant une caractéristique préférée, l'huile essentielle provient de la plante fournissant l'extrait. Elle est de préférence obtenue par distillation, notamment distillation à la vapeur. Lorsque la même plante ou le même matériel végétal est employé comme source de l'huile essentielle et de l'extrait, l'huile est extraite avant la production de l'extrait proprement dit.

Suivant une caractéristique préférée de l'invention, l'huile essentielle et l'extrait proviennent *d'Alpinia galanga*. On peut ainsi employer l'huile essentielle « Galanga root essential oil » obtenue par distillation à la vapeur de racines sèches et commercialisée par EVAR, Extraction Végétale et Aromatique, Félines, 49320 Coutures, France.

Dans la présente invention, les dérivés de phénylpropène répondent à la formule (I) suivante : 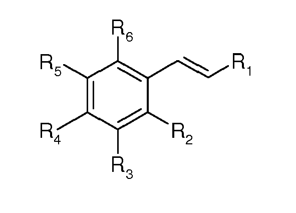 dans laquelle :
- R₂, R₃, R₄, R₅ et R₆ représentent indépendamment l'un de l'autre un groupe hydroxyle, un atome d'hydrogène ou le radical -O-R₇, ou -O-C(O)-R₇ dans lesquels R₇ représente un groupe alkyle en C₁-C₃₀, avantageusement en C₁-C₆, de préférence en C₁ ;
- R₁ représente un groupe C(OH), un groupe -C(O)OH ou un groupe -C-O-C(O)-R₇ dans lequel R₇ représente un groupe alkyle en C₁-C₃₀, avantageusement en C₁-C₆, de préférence en C₁.

Parmi les dérivés de phénylpropène, on citera tout particulièrement : les acétates et diacétates, les groupes acétates pouvant être portés par le phényl et/ou par le radical propène, par exemple le para-diacétate de coumaryle et le para-acétate de coumaryle ; et les dérivés de phénylpropénol exempts de groupe acétate, par exemple l'alcool coniférylique et l'alcool cinnamique. On peut aussi mentionner l'acide caféique, l'alcool p-méthoxy-trans-cynnamylique, l'alcool 3,4-diméthoxy-trans-cinnamylique.

Dans la présente invention, par dérivé de type chavicol on entend notamment toute molécule répondant à la formule générale suivante : 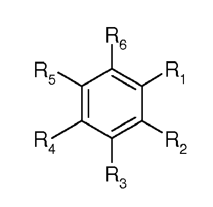 dans laquelle R₂, R₃, R₄, R₅ et R₆ représentent indépendamment l'un de l'autre un groupe hydroxyle, un atome d'hydrogène ou le radical -O-R₇, ou -O-C(O)-R₇ dans lesquels R₇ représente un groupe alkyle en C₁-C₃₀ ;
R₁ représente un radical de formule : 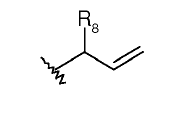 dans lequel R₈ représente notamment un groupe hydroxyle ou le radical -O-C(O)-R₉ dans lequel R₉ représente un groupe alkyle en C₁-C₆, ou éventuellement un atome d'hydrogène

Comme exemple de dérivé de type chavicol on citera notamment l'acétate de 1'-acetoxychavicol :

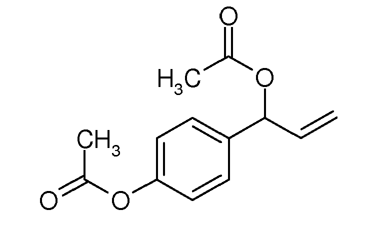

Suivant un mode de réalisation préféré la composition comprend un terpène ou un terpénoïde et un extrait d'une plante de la famille des Zingiberaceae, par exemple *Alpinia galanga,* contenant des dérivés acétates et/ou diacétates de phénylpropène et des dérivés de phénylpropénol dans un pourcentage en poids par rapport au poids total de l'extrait sec supérieur à environ 15%, notamment supérieur à environ 20%, en particulier supérieur à environ 30%, voire supérieur à environ 40%. Ce pourcentage peut notamment être compris entre environ 20 et environ 60%.

Dans un mode de réalisation préféré de l'invention la composition contient d'environ 10 à environ 40%, notamment d'environ 15 à environ 40%, notamment d'environ 20 à environ 40% de dérivé(s) diacétate et/ou acétate de phénylpropène, les % étant exprimés en poids par rapport au poids total de l'extrait sec.

Dans un autre mode de réalisation préféré de l'invention la composition contient d'environ 5 à environ 20%, notamment d'environ 10 à environ 20% de dérivé (s) de phénylpropénol, les % étant exprimés en poids par rapport au poids total de l'extrait sec.

Dans un mode de réalisation encore plus préféré, la composition contient d'environ 10 à environ 40%, notamment d'environ 15 à environ 40%, notamment d'environ 20 à environ 40% de dérivé(s) diacétate et/ou acétate de phénylpropène et d'environ 5 à environ 20% , notamment d'environ 10 à environ 20% de dérivé(s) de phénylpropénol, les % étant exprimés en poids par rapport au poids total de l'extrait sec.

Dans un mode de réalisation encore plus préféré, la composition contient d'environ 10 à environ 40%, notamment d'environ 15 à environ 40%, notamment d'environ 20 à environ 40% de dérivés diacétate et acétate de phénylpropène et d'environ 5 à environ 20%, notamment d'environ 10 à environ 20% de dérivé(s) de phénylpropénol, les % étant exprimés en poids par rapport au poids total de l'extrait sec.

La teneur initiale en dérivés de type chavicol dans la plante de départ conditionne la quantité totale de dérivés acétates et/ou diacétates de phénylpropène et de dérivés de phénylpropénol que l'on peut obtenir par extraction et réarrangement conformément à l'invention. On peut toutefois ajuster les teneurs dans le produit final, ou encore augmenter les teneurs au-delà des valeurs décrites ci-dessus, en purifiant ou en concentrant, comme il sera décrit plus loin.

Suivant une caractéristique de ces divers modes de réalisation, la composition comprend des dérivés acétate et des dérivés diacétate, la somme des deux dans les proportions indiquées.

Dans un mode de réalisation préféré, ces acétates et diacétates sont l'acétate, notamment le para-acétate de coumaryle et le diacétate, de préférence le para-diacétate de coumaryle. De préférence, la composition contient les deux espèces, notamment para-acétate de coumaryle et para-diacétate de coumaryle.

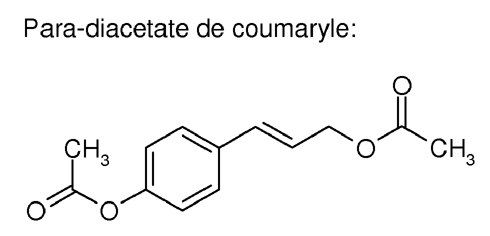

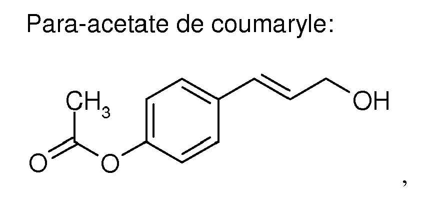

Dans un mode de réalisation préféré, les dérivés de phénylpropénol sont choisis parmi l'alcool coniférylique et d'alcool cinnamique.

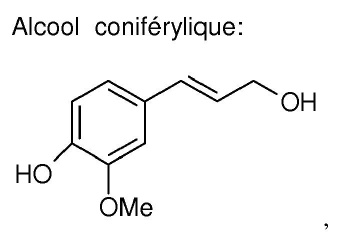

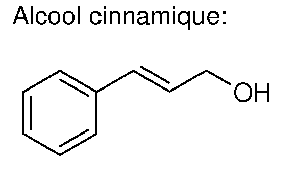

Suivant une modalité de l'invention, la composition comprend au moins un terpène ou un terpénoïde et un extrait d'une plante de la famille des Zingiberaceae, susceptible d'être obtenu à partir de matière végétale de la famille des Zingiberaceae, notamment Alpinia galanga, par un procédé d'extraction comprenant une étape de macération en présence d'un solvant et une étape dans laquelle l'extrait issu de la macération est soumis au reflux d'une solution aqueuse dans des conditions, notamment de durée et de température, conduisant à l'obtention d'un extrait contenant des dérivés phénylpropène et étant exempt des dérivés de type chavicol. D'autres caractéristiques du procédé de préparation de l'extrait et des terpènes et terpénoïdes sont données plus loin.

L'absence de dérivés de type chavicol peut être contrôlée par Chromatographie Liquide Haute Performance (CLHP ou HPLC) ou par chromatographie gazeuse (CG) couplée à un spectromètre de masse (SM) en suivant par exemple le mode opératoire décrit dans la partie exemple. De même, la nature et la teneur en dérivés acétate et diacétate de phénylpropène et en dérivés phénylpropénol peuvent être obtenus par cette méthode.

L'invention a aussi pour objet un produit pharmaceutique ou un complément alimentaire comprenant une composition selon l'invention.

Suivant une caractéristique, la quantité d'extrait dans le produit ou complément est comprise entre environ 100 mg et environ 400 mg par unité de dose, de préférence entre environ 150 mg et 300 mg par unité de dose.

Suivant une autre caractéristique, la quantité de terpène et/ou terpénoïde, e.g. d'huile essentielle, est comprise entre environ 5 et 40 mg, de préférence entre 5 et 30 mg par unité de dose.

Ce produit ou complément peut avantageusement se présenter sous une forme pour ingestion orale ou sous forme topique. Parmi les formes pour ingestion orale, on peut citer notamment les suspensions buvables comme les sirops et les formes sèches comme des tablettes, des pastilles, des gélules, des comprimés, des capsules molles ou dures, des gommes à mâcher. Parmi les formes topiques, on peut citer les formes liquide ou solide pour pulvérisation ou spray, les formes d'application manuelle telles que les pommades, les patchs, les gels. Ces formes topiques sont destinées à une application dans la bouche, notamment sur le voile du palais, ou dans la fosse nasale ou sur la muqueuse nasale. On n'exclut pas non plus une administration par voie parentérale.

L'homme de l'art dans le domaine de la galénique saura mettre en oeuvre les différentes formes utiles pour l'administration des produits et/ou compléments de l'invention.

Le produit pharmaceutique ou complément alimentaire peut ainsi contenir tout additif utile pour la mise sous forme. Il peut ainsi contenir au moins un excipient. Parmi les excipients pour forme liquide, on citera la glycérine et le miel et pour forme solide, la maltodextrine, la silice, le stéarate de magnésium.

Il peut en outre comporter tout autre additif utile, tel qu'un promoteur de pénétration dans la muqueuse pour les formes topiques.

La présente invention a aussi pour objet une telle composition ou un tel produit pharmaceutique ou complément alimentaire pour utilisation comme médicament pour la prévention, la diminution ou la suppression de l'apnée du sommeil.

La présente invention a aussi pour objet une telle composition ou un tel produit pharmaceutique pour utilisation comme médicament pour la prévention, la diminution ou la suppression du ronflement.

L'invention a aussi pour objet une méthode de traitement et/ou de prévention du ronflement et/ou de l'apnée du sommeil, dans lequel on administre à un patient une composition, un produit pharmaceutique ou un complément alimentaire selon l'invention.

L'un des avantages de la présente invention réside dans le mode d'application, qui ne se limite pas à la voie nasale, mais peut aussi prendre la forme d'une administration orale. L'utilisation d'une forme orale est rendue possible par l'absence de composés agressifs pour l'estomac. Ceci confère un avantage important à l'invention. En effet, les applications topiques répétées sur les muqueuses peuvent s'avérer irritantes à la longue. Le fait de disposer de formes orales efficaces permet d'éviter cet inconvénient. Il est ainsi possible de combiner les deux formes d'administration orale et topique.

La dose journalière des produits, compléments ou compositions selon l'invention peut varier selon les besoins et la gravité des symptômes du patient. Typiquement, la dose journalière se situe entre environ 300 mg et environ 1 g de l'extrait, par exemple de l'extrait *d'Alpinia galanga* préparé précédemment, de préférence entre environ 400 mg et environ 800 mg.

L'administration se fera de préférence le matin ou en début d'après-midi (i.e. jusqu'à 14 ou 15 heures (2 ou 3 pm).

La présente invention a aussi pour objet un procédé d'obtention d'un extrait de Zingiberaceae comprenant une étape de macération de matière végétale de la famille des Zingiberaceae, notamment *Alpinia galanga,* en présence d'un solvant et une étape dans laquelle l'extrait issu de la macération est soumis au reflux d'une solution aqueuse dans des conditions opératoires, notamment de durée et de température, conduisant à l'obtention d'un extrait exempt de dérivés de type chavicol et contenant d'environ 10 à environ 40%, notamment d'environ 15 à environ 40%, notamment d'environ 20 à environ 40% de dérivé acétate et/ou diacétate de phénylpropène, et d'environ 5 à environ 20%, notamment d'environ 10 à environ 20% de dérivés de phénylpropénol, les % étant exprimés en poids par rapport au poids total de l'extrait sec. Suivant une caractéristique, l'extrait comprend des dérivés acétate et des dérivés diacétate, la somme des deux dans les proportions indiquées.

La présente invention a aussi pour objet un procédé de préparation d'une composition selon l'invention comprenant au moins un terpène ou un terpénoïde, notamment une huile essentielle, et un extrait d'une plante de la famille des Zingiberaceae, l'extrait contenant des dérivés de phénylpropène, et étant exempt de dérivés de type chavicol. La préparation de l'extrait ou procédé d'extraction proprement dit comporte (a) une étape de macération de matière végétale de la famille des Zingiberaceae, notamment *Alpinia galanga,* en présence d'un solvant et une étape dans laquelle l'extrait issu de la macération est soumis au reflux d'une solution aqueuse dans des conditions opératoires, notamment de durée et de température, conduisant à l'obtention d'un extrait contenant des dérivés de phénylpropène, et étant exempt de dérivés de type chavicol, et (b) l'addition à cet extrait d'un terpène ou terpénoïde, notamment d'une huile essentielle.

Ces deux procédés peuvent en outre comprendre ou être précisés par, une, plusieurs ou l'ensemble des caractéristiques qui sont exposés ci-après.

L'étape de reflux selon l'invention conduit à un réarrangement sigmatropique des dérivés de phénylpropène et est conduite dans des conditions adaptées à l'obtention d'une composition contenant les espèces chimiques souhaitées dans les proportions souhaitées.

Le terpène ou terpénoïde peut provenir d'une source commerciale ou provenir de la distillation d'une plante sèche ou fraîchement coupée. De préférence l'huile essentielle est produite à partir de la même matière végétale avant de mettre en oeuvre le procédé d'extraction, ou à partir d'un autre lot de cette matière végétale. Avantageusement, on utilise le rhizome. Une source commerciale a été indiquée supra.

La quantité d'huile essentielle peut simplement être ajustée pour reproduire sensiblement son pourcentage dans la plante servant à produire l'extrait.

L'obtention de l'huile essentielle peut être réalisée par les techniques de distillation usuelles. Parmi elles, on retiendra plus particulièrement la distillation par entraînement à la vapeur d'eau et la distillation par utilisation de CO₂ supercritique. On emploie de préférence la distillation par entraînement à la vapeur d'eau.

La matière végétale pour l'étape de macération peut être la plante entière ou seulement, et de préférence seulement, les rhizomes. Cette matière végétale est utilisée de préférence sous forme sèche. La matière végétale fraiche peut notamment être lyophilisée juste avant usage.

Le solvant utilisé lors de l'étape de macération de l'invention peut être tout solvant d'extraction classique pour préparer des extraits de plantes destinés à une consommation humaine.

Le solvant est de préférence un solvant alcoolique. Il s'agit avantageusement d'un alcool à courte chaîne, notamment dont la chaîne carbonée comprend de 1 à 4 carbones. Parmi les alcools on citera le méthanol et l'éthanol. L'éthanol est le solvant préféré. Le solvant alcoolique a un titre en alcool avantageusement compris entre environ 90 et environ 98%, de préférence entre environ 95 et 98%.

Dans certains cas, on peut aussi utiliser de l'alcool à un titre inférieur, par exemple de 30%

Le solvant peut aussi être de l'acétone ou l'éther éthylique.

Des mélanges de ces solvants peuvent aussi être employés.

Lorsque l'on utilise un mélange d'alcool contenant de l'éthanol et au moins un autre alcool, par exemple du méthanol, la proportion d'éthanol est avantageusement supérieure à environ 60%, préférentiellement supérieure à environ 70%, plus préférentiellement supérieure à environ 80%, encore plus préférentiellement supérieure à environ 90%, et jusqu'à environ 98%, par rapport au mélange des alcools.

Lors de l'étape de macération, la quantité en poids de solvant est supérieure au poids de matière végétale. Avantageusement, le rapport en poids solvant : matière végétale est compris entre 1 : 1,5 et 1 : 4. Il est typiquement de 1 : 2.

La durée de l'étape de macération doit être suffisante pour extraire le maximum des extractibles. Typiquement, cette étape a une durée d'environ 24 à 72 heures, préférentiellement d'environ 48 heures.

La macération est conduite avantageusement à température ambiante (20 à 25°C) ou à une température comprise entre environ 20 et environ 25.°C.

Lorsque l'on conduit plusieurs opérations de macération successives les solutions d'extraction obtenues sont rassemblées.

La solution d'extraction (provenant d'une étape unique ou du rassemblement de plusieurs solutions) est ensuite filtrée, de manière à éliminer la matière solide et récupérer un filtrat.

Le solvant est ensuite éliminé. Par exemple, le filtrat est concentré, par exemple sous vide et à température adaptée (par exemple entre environ 40 et environ 60°C, typiquement aux alentours de 50°C. On peut par exemple employer un rotavapeur.

Suivant une caractéristique de l'invention, l'étape de reflux est conduite sur un extrait de macération qui est concentré notamment par élimination totale ou partielle du solvant.

A ce stade, on peut si on le souhaite concentrer les dérivés de type chavicol. Les techniques usuelles telles que la chromatographie sur colonne HPLC (par exemple sur silice) ou l'extraction sélective (par exemple extraction liquide/liquide, avec un solvant aqueux et un solvant tel que dichlorométhane ou acétate d'éthyle) peuvent être employées.

Le produit ainsi obtenu, est ensuite additionné d'eau et soumis à un reflux dans un montage ou une installation à reflux.. Le rapport eau : produit peut être compris entre 1 :1 et 1 :2.

Le mélange hétérogène ainsi obtenu est porté au reflux pendant une durée et à une température conduisant à l'obtention d'un extrait conforme à l'invention.

Ces conditions de reflux sont de préférence choisies pour l'obtention d'un extrait exempt de dérivés de type chavicol et contenant d'environ 10 à environ 40%, notamment d'environ 15 à environ 40%, notamment d'environ 20 à environ 40% de dérivés acétate et/ou diacétate de phénylpropène et d'environ 5 à environ 20%, notamment d'environ 10 à environ 20% de dérivés de phénylpropénol, les % étant exprimés en poids par rapport au poids total de l'extrait sec. Suivant une caractéristique, l'extrait obtenu comprend à la fois des dérivés acétate et des dérivés diacétate, la somme des deux dans les proportions indiquées.

Dans un mode de réalisation préféré de l'invention la température du mélange est comprise entre environ 100°C et environ 130°C, de préférence entre environ 100°C et environ 120°C. Le reflux est maintenu pendant une durée adéquate. Cette durée est notamment supérieure ou égale à 1 heure et avantageusement, elle est comprise entre environ 1 heure et environ 3 heures.

Le solvant aqueux est ensuite évaporé pour conduire à l'extrait final.

On peut si on le souhaite concentrer les dérivés obtenus. Les techniques usuelles telles que la chromatographie sur colonne HPLC (par exemple sur silice) ou l'extraction sélective (par exemple extraction liquide/liquide, avec un solvant aqueux et un solvant tel que dichlorométhane ou acétate d'éthyle) peuvent être employées. Cette étape peut notamment être conduite après évaporation du solvant aqueux.

L'extrait ainsi obtenu est mélangé à un terpène ou un terpénoïde, de préférence une huile essentielle, comme décrit ci-dessus. Les éventuels excipients et additifs sont aussi ajoutés.

Un autre objet de l'invention est un extrait d'*Alpinia galanga* obtenu par le procédé selon l'invention décrit ci-dessus caractérisé en ce qu'il contient des dérivés de phénylpropène et qu'il est exempt de dérivés de type chavicol.

Un autre objet de l'invention est un extrait d'*Alpinia galanga* qui est exempt de dérivés de type chavicol et contient d'environ 10 à environ 40%, notamment d'environ 15 à environ 40%, notamment d'environ 20 à environ 40% de dérivés acétate et/ou diacétate de phénylpropène, les % étant exprimés en poids par rapport au poids total de l'extrait sec.

Un autre objet de l'invention est un extrait d'*Alpinia galanga* qui est exempt de dérivés de type chavicol et contient d'environ 5 à environ 20%, notamment d'environ 10 à environ 20% de dérivés de phénylpropénol, les % étant exprimés en poids par rapport au poids total de l'extrait sec et.

Un autre objet de l'invention est un extrait d'*Alpinia galanga* qui est exempt de dérivés de type chavicol et contient d'environ 10 à environ 40%, notamment d'environ 15 à environ 40%, notamment d'environ 20 à environ 40% de dérivés acétate et/ou diacétate de phénylpropène et d'environ 5 à environ 20%, notamment d'environ 10 à environ 20% de dérivés de phénylpropénol, les % étant exprimés en poids par rapport au poids total de l'extrait sec.

Un autre objet de l'invention est un extrait d'*Alpinia galanga* qui est exempt de dérivés de type chavicol et contient d'environ 10 à environ 40%, notamment d'environ 15 à environ 40%, notamment d'environ 20 à environ 40% de dérivés acétate et diacétate de phénylpropène et d'environ 5 à environ 20%, notamment d'environ 10 à environ 20% de dérivés de phénylpropénol, les % étant exprimés en poids par rapport au poids total de l'extrait sec.

Dans un mode de réalisation préféré de ces extraits d'Alpinia galanga, les acétates et diacétates sont l'acétate, notamment le para-acétate de coumaryle et le diacétate, de préférence le para-diacétate de coumaryle, et les dérivés de phénylpropénol sont choisis parmi l'alcool coniférylique et d'alcool cinnamique.

Les exemples suivants de préparation d'extraits sont donnés uniquement à titre d'illustration de l'objet de l'invention dont ils ne constituent en aucune manière une limitation.

### EXEMPLES :

Techniques d'analyse :

### Méthode 1 : détection d'acétate de 1'- acétoxychavicol (AAC).

Les produits sont analysés par HPLC Agilent 1100 équipé d'un détecteur UV à barrette de diode, couplé à un spectromètre de masse.

Caractéristiques de la chromatographie liquide à haute performance :
- colonne Spérisorb ODS 2, 3µ, diamètre : 5 mm , longueur : 250 mm.
- débit à 0,6 ml / min
- Température de la colonne : 30°C
- Gradient de la phase mobile : un mélange d'eau (A), acétonitrile (B) (A : 10, B : 90 → A : 0, B : 100) en 45 min
- Echantillons solubilisés dans le méthanol, injection de 1 µl
- Longueur d'onde de détection : 220 nm ou 253 nm.

Signature de la présence d'AAC :1 pic au temps de rétention de 22,41 mn, avec un UV max de 221 nm, avec un fragment en positif de 132.

### Méthode 2 : Suivi de la transformation de l'AAC en dérivés acétate et/ou diacétate de phénylpropène et dérivés de phénylpropénol.

Ce suivi est effectué au moyen d'une technique d'analyse couplée à un détecteur de spectrométrie de masse (SM). La spectrométrie de masse est effectuée soit après une chromatographie liquide haute performance AGILENT 1100 (ci-dessus) avec une source APCI ou electrospray, soit en utilisant une chromatographie gazeuse (CG) HEWLETT PACKARD 6890, avec un détecteur de masse 5973 H.P.

Les données de la spectrométrie de masse sont exprimées en masse/nombre de charge (m/z). Les valeurs d'intensité des fragments sont référencées par rapport au pic le plus intense, auquel on attribue une intensité de 100%.
* Analyse en chromatographie gazeuse Hewlett Packard 6890 avec un détecteur de masse 5973 HP.
   - colonne apolaire ( 5% diphényl et 95 % diméthylpolysiloxane), HP-5-MS
   - température initialement à 40 °C pendant 6 min, augmentation jusqu'à 300 °C, avec 5° C par min
      température de l'injecteur 200° C.
   - échantillons solubilisés dans le méthanol, injection de 1 µl.

Signatures :

| Produits | Temps de rétention | Pic Moléculaire | Masse des fragments par (GC/SM) |
|---|---|---|---|
| AAC | 13,13mn | 234 | 192, 170, 150, 132, 121. |
| Para-diacétate de coumaryle | 14,74mn | 234 | 192, 149, 133, 121, 107, 94, 77. |
| Para-acétate de coumaryle | 14,04 mn | 192 | 150, 131, 121, 107, 94, 77. |

Pour caractériser ou déterminer la présence d'un composé particulier, une méthode consiste à déterminer au préalable sa signature à partir du composé pur. Ainsi, l'alcool coniférylique et l'alcool cinnamique sont disponibles commercialement. Une fois leur signature obtenue, on peut les détecter et mesurer leur quantité dans l'extrait réarrangé.

### Exemple 1: Extraction de l'huile essentielle terpénique.

50 Kg de rhizomes frais coupés d'Alpinia galanga sont introduits dans un alambic, puis le système est mis en route. Après extraction et décantation de la phase supérieure, on obtient une huile terpénique jaune odorante représentant 1,7-1,9% en poids par rapport au poids de rhizomes de départ.

### Exemple 2: Extrait d'un mélange de dérivés de phénylpropène conforme à l'invention.

La racine résiduelle d'Alpinia galanga issue de l'exemple 1 a été déposée sur des plateaux, qui ont par la suite été placés dans un lyophilisateur pendant une durée de 72 heures. On a obtenu un poids total de sortie de 47 kg.

45 kg de racine lyophilisée obtenus ont été additionnés de 90 kg d'éthanol à 95-98%. Après 48 heures de macération, le mélange hétérogène a été filtré sur tamis de 100 microns et la solution obtenue stockée dans un récipient. La matière végétale résiduelle a été de nouveau remise en macération dans les mêmes conditions décrites précédemment. Cette opération a été renouvelée une dernière fois.

Les solutions obtenues ont été rassemblées, puis filtrées sur tamis de 40 microns et enfin concentrées sous pression réduite pour conduire à 3 kg d'une pâte visqueuse marron odorante. Le rendement était de 6,66%. A 2 kg de l'extrait d'Alpinia galanga obtenu précédemment ont été additionnés 2 kg d'eau. Le mélange hétérogène ainsi obtenu a été porté au reflux de l'eau à une température légèrement supérieure à 100° C pendant 2h30 dans une cuve en inox surmontée d'un réfrigérant. Le solvant aqueux a ensuite été évaporé sous pression réduite à une température de l'ordre de 50°C, pour conduire à un extrait d'une masse de 1,9 kg.
L'extrait a été soumis à une analyse par spectrométrie de masse CG/SM, et l'on a ainsi pu vérifier l'absence d'acétoxyacétate de chavicol (AAC).

De la même manière, on a dosé les dérivés de phénylpropène et les résultats sont rassemblés dans le tableau ci-dessous :

| Produits/ T °C et durée | Extrait *A. galanga* % AAC | Para diacétate de coumaryle | Para acétate de coumaryle | Alcool coniférylique et alcool cinnamique |
|---|---|---|---|---|
| 0°C | 38.5% | 1% | 0.8% | - |
| 10 °C 2h30 | 0% | 12,95% | 5,50% | 8,50% |

La proportion des produits para et diacétates de coumaryle dépendent essentiellement de la teneur en AAC dans l'extrait initial d'*Alpinia galanga*

## Revendications

1. Composition comprenant au moins un terpène ou un terpénoïde et un extrait d'une plante de la famille des Zingiberaceae, de préférence choisie parmi les espèces *Alpinia galanga, Alpinia officinarum, Zingiber officinalis, Zingiber cassumunar* et *Zingiber zerumbet,* de préférence *Alpinia galanga,* l'extrait contenant des dérivés de phénylpropène et étant exempt de dérivés de type chavicol.

2. Composition selon la revendication 1, comprenant une huile essentielle, de préférence l'huile essentielle provient d'*Alpinia galanga.*

3. Composition la revendication 1 ou 2, dans laquelle l'extrait contient des dérivés acétate et/ou diacétate de phénylpropène et des dérivés de phénylpropénol.

4. Composition selon la revendication 3, dans laquelle l'extrait contient de 10 à 40%, notamment de 15 à 40%, de préférence de 20 à 40% de dérivé(s) diacétate et/ou acétate de phénylpropène et/ou de 5 à 20%, notamment de 10 à 20% de dérivé(s) de phénylpropénol, les % étant exprimés en poids par rapport au poids total de l'extrait sec.

5. Composition selon l'une quelconque des revendications précédentes, dans laquelle l'extrait contient des composés choisis parmi le groupe comprenant le para-diacétate de coumaryle, le para-acétate de coumaryle, l'alcool coniférylique et l'alcool cinnamique.

6. Composition selon la revendication 5, dans laquelle l'extrait contient de 10 à 40%, notamment de 15 à 40%, de préférence de 20 à 40% d'acétate et/ou diacétate de coumaryle, et/ou de 5 à 20%, notamment de 10 à 20% d'alcool coniférylique et/ou d'alcool cinnamique, les % étant exprimés en poids par rapport au poids total de l'extrait sec.

7. Composition selon l'une quelconque des revendications précédentes, dans laquelle le terpène ou le terpénoïde provient de l'huile essentielle de cardamome ou d'une plante de la famille des Zingiberaceae choisie parmi les espèces *Alpinia galanga, Alpinia officinarum, Zingiber officinalis, Zingiber cassumunar* et *Zingiber zerumbet.*

8. Produit pharmaceutique ou complément alimentaire comprenant une composition selon l'une quelconque des revendications précédentes.

9. Produit ou complément selon la revendication 8, dans lequel la quantité d'extrait est comprise entre 100 et 400 mg, de préférence entre 150 et 300 mg par unité de dose et/ou la quantité de terpène et/ou terpénoïde, e.g. d'huile essentielle, est comprise entre 5 et 40 mg, de préférence entre 5 et 30 mg par unité de dose.

10. Produit ou complément selon les revendications 8 ou 9, pour utilisation comme médicament pour la prévention, la diminution ou la suppression de l'apnée du sommeil et/ou du ronflement.

11. Procédé d'obtention d'un extrait à partir de matière végétale de la famille des Zingiberaceae de préférence choisie parmi les espèces *Alpinia galanga, Alpinia officinarum, Zingiber officinalis, Zingiber cassumunar* et *Zingiber zerumbet,* comprenant une étape de macération en présence d'un solvant, puis l'extrait issu de la macération est soumis au reflux d'une solution aqueuse pendant une durée et à une température conduisant à l'obtention d'un extrait exempt de dérivés de type chavicol et contenant de 10 à 40%, notamment de 15 à 40%, de préférence de 20 à 40% de dérivé(s) diacétate et/ou acétate de phénylpropène et/ou de 5 à 20%, notamment de 10 à 20% de dérivé(s) de phénylpropénol, les % étant exprimés en poids par rapport au poids total de l'extrait sec.

12. Procédé selon la revendication 11, dans lequel la durée du reflux est comprise entre 1 et 3 heures et la température est comprise entre 100°C et 130°C, préférentiellement entre 100 et 120°C.

13. Procédé de préparation d'une composition selon l'une quelconque des revendications 1 à 7, dans lequel l'extrait est obtenu par un procédé d'extraction comprenant une étape de macération en présence d'un solvant, puis l'extrait issu de la macération est soumis au reflux d'une solution aqueuse, puis le terpène et/ou terpénoïde, de préférence l'huile essentielle, est ajouté à l'extrait.

14. Extrait, notamment extrait d'*Alpinia galanga,* susceptible d'être obtenu par la mise en oeuvre du procédé selon l'une quelconque des revendications 11 à 13.
